(19) Europäisches Patentamt
European Patent Office
Office européen des brevets

(11) Veröffentlichungsnummer: **0 002 485**
**B1**

(12) # EUROPÄISCHE PATENTSCHRIFT

(45) Veröffentlichungstag der Patentschrift: **28.04.82**

(51) Int. Cl.³: **A 61 K 43/00**

(21) Anmeldenummer: **78101563.1**

(22) Anmeldetag: **05.12.78**

(54) **Verfahren zur Herstellung zinnhaltiger Markierungsbestecke.**

(30) Priorität: **15.12.77 DE 2755874**

(43) Veröffentlichungstag der Anmeldung:
**27.06.79 Patentblatt 79/13**

(45) Bekanntmachung des Hinweises auf die Patenterteilung:
**28.04.82 Patentblatt 82/17**

(84) Benannte Vertragsstaaten:
**BE DE FR GB IT**

(56) Entgegenhaltungen:
**DE - A - 2 543 350**
**GB - A - 1 429 549**

Die Akte enthält technische Angaben die nach dem Eingang der Anmeldung eingereicht wurden und die nicht in dieser Patentschrift enthalten sind.

(73) Patentinhaber: **HOECHST AKTIENGESELLSCHAFT**
**Postfach 80 03 20**
**D-6230 Frankfurt/Main 80 (DE)**

(72) Erfinder: **Schwarz, Alexander, Dr.**
**Wiesenring 61**
**D-6093 Flörsheim am Main (DE)**

**EP 0 002 485 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung zinnhaltiger Markierungsbestecke

Technetium-99m-markierte Pyrophosphate bzw. Polyphosphate werden in der Nuklearmedizin zur Knochenszintigraphie verwendet. Nach DE—A—26 06 561 ist Technetium-99m-Zinn-Pyrophosphat als Knochendiagnostikum beschrieben. Die Markierungseinheit ist ein Gemisch aus den Komponenten Natriumpyrophosphat und Zinn-II-chlorid, dessen wäßrige Lösung nach Einstellung auf einen pH-Bereich von 3 bis 9 lyophilisiert wird.

Durch Zugabe von Natriumpertechnetat-99m-Tc-Lösung (z.B. aus einem Technetium-99m-Generator) wird die Markierungseinheit mit dem $\delta$-Strahler Tc-99m markiert. Das zweiwertige Zinn im Zinn-II-chlorid reduziert das Pertechnetat zu $Tc^{4+}$ oder zu einer niedrigeren Wertigskeitsstufe, und dieses reduzierte Tc-99m wird vom Pyrophosphat gebunden. Nach intravenöser Injektion lagert sich das radioaktiv markierte Pyrophosphat an die Hydroxyapatitkristalle der Knochen an, bzw. es wird nach Spaltung durch Pyrophosphatase in die Knochen eingebaut. Es kann so zur szintigraphischen Skelettdarstellung benutzt werden.

Ähnlich verhalten sich Diphosphonate, bei denen der Sauerstoff, der im Pyrophosphat (—P—O—P—) die Phosphoratome verbindet, durch ein Kohlenstoffatom

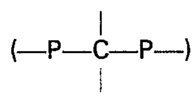

ersetzt ist. 1-Hydroxyäthan-1,1-diphosphonsäure (HEDP) der Formel

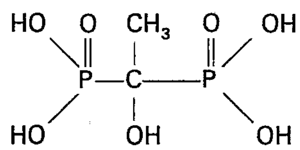

markiert mit Tc—99m, zeichnet sich gegenüber den Pyro-bzw. Polyphosphaten vor allem durch eine bedeutend schnellere Plasmaclearance aus, was bei der szintigraphischen Abbildung zu einem besseren Kontrast zwischen Knochen- und Weichteilaktivität führt (J. Nucl. Med. *13* (1972), 947).

Die einfachste Diphosphonsäure, Methylendiphosphonsäure (MDP), der Formel

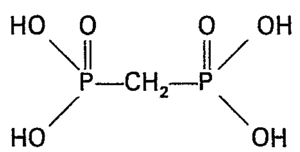

zeigt die bisher günstigsten Eigenschaften als knochenaffine Substanz, da ihre Anreicherung im Skelett bereits nach zwei Stunden das Optimum erreicht, so daß bei gleicher Bildqualität im Vergleich zu den Phosphaten und dem HEDP eine frühere Knochenszintigraphie möglich ist (J. Nucl. Med. *14* (1973), 640).

Inzwischen wurde eine Reihe von Tc-99m-Markierungsbestecken, die Methylendiphosphonsäure als knochenaffine Trägersubstanz enthalten, beschrieben. Sie unterscheiden sich durch die verschiedenen Zinn-II-salze, die sie als Reduktionsmittel für das Pertechnetat enthalten.

So wird nach DE—A—2 424 496 und nach GB—A—1 429 549 $SnCl_2 \cdot 2\ H_2O$ verwendet. Als reduzierend wirkende Komponenten von Markierungseinheiten auf Basis Methylendiphosphonsäure sind ferner bekannt: Zinn-II-fluorid, Zinn-II-tartrat (US-Patent 3 987 157) und Zinn-II-pyrophosphat (DE—A—2 534 985).

Die bekannten Markierungsbestecke bestehen aus einem Gemisch der knochenaffinen Diphosphonsäure mit einem der genannten Zinn-II-salze. Weiterhin werden substituierte Diphosphonsäuren zu diesem Zweck verwendet, wie aus DE—A—25 43 350 bekannt ist.

Die vorliegende Erfindung betrifft ein Verfahren zur Herstellung von Markierungsbestecken, die das Zinn-II-salz einer Diphosphonsäure enthalten, deren beide Phosphorsäurereste an demselben Kohlenstoff gebunden sind.

Das Verfahren zur Herstellung eines zur Markierung mit Technetium-99m geeigneten zinnsalzhaltigen Gemisches (Markierungsbesteck) ist dadurch gekennzeichnet, daß man Zinn-II-oxid entweder

a) mit dem Überschuß einer Natrium-diphosphonatlösung, die einen pH-Wert zwischen 6,2 und 7,2 aufweist, umsetzt, oder
b) mit einem Überschuß an Natronlauge löst und soviel Diphosphonsäure zugibt, bis ein pH-Wert zwischen 6,2 und 7,2 erreicht ist.

In jedem Fall erhält man ein lösliches, auf Pertechnetat reduzierend wirkendes Zinn-II-diphosphonat.

Die Umsetzung des Zinn-II-oxids nach den beiden Verfahrensvarianten a) und b) erfolgt mit dem jeweiligen Reaktionspartner bei Temperaturen zwischen 20 und 100°C, vorzugsweise bei Raumtemperatur.

Als Diphosphonsäuren kommen erfindungsgemäß vorzugsweise die Methylendiphosphonsäure, 1,1-Diphosphonopropan-2,3-dicarbonsäure und 1,1-Diphosphonobutan-3,4-dicarbonsäure in Betracht.

Bei dem Verfahren wird die Diphosphonsäure gegenüber dem Zinn im Überschuß eingesetzt. Es ist in diesem Fall ausreichend, wenn jedes zehnte bis hunderste, vorzugsweise etwa jedes 30.—50. Diphosphonsäure-Molekül ein Mono- Zinn-II-diphosphonat bildet. Dies entspricht einem Zinngehalt von 1 bis 3 Gewichtsprozent, berechnet auf Diphosphonsäure, wobei das Zinn als Zinn-II-salz der Diphosphonsäure vorliegt. Die erhaltenen Produkte zeichnen sich durch einen im Vergleich zu den bekannten Markierungsbestecken niedrigeren Zinngehalt aus.

Die Zinn-II-diphosphonate sind in Gegenwart eines Überschusses an freier Diphosphonsäure nur begrenzt haltbar, da sie zu Komplexsalzen des vierwertigen Zinns weiterreagieren, wodurch ihre Reduktionsfähigkeit gegenüber Pertechnetat verlorengeht.

Aus diesem Grunde wird in der vorliegenden Erfindung nach der Verfahrensvariante a) durch vorherige Zugabe von Natronlauge zur wässrigen Lösung der Diphosphonsäure zuerst ein pH-Bereich von 6,2 bis 7,2, vorzugsweise 6,5, eingestellt und dann erst Zinn-II-oxid zugesetzt, wobei das stabile Natrium-Zinn-II-salz der Diphosphonsäure entsteht.

Zum gleichen Salz gelangt man auch nach Verfahrensvariante b), wenn Zinnoxid in Natronlauge gelöst und die dabei entstehende Natriumstannitlösung mit so viel in Wasser gelöster Diphosphonsäure versetzt wird, bis sich ein pH-Wert von 6,2 bis 7,2, vorzugsweise 6,5, eingestellt hat.

Voraussetzung für die einwandfreie Herstellung des Natrium-Zinn-II-salzes der Diphosphonsäure ist bei beiden Methoden der sorgfältige Ausschluß von Sauerstoff während der Umsetzung.

Die so erhaltenen Lösungen reduzieren zugesetztes Natriumpertechnetat ($^{99m}TcO_4^-$) praktisch quantitativ, wobei das 3- bzw. 4-wertige Technetium vom Diphosphonat, vorzugsweise dem Dinatriumsalz der Diphosphonsäure, salzartig gebunden wird.

Um die Lösungen, welche die Zinn-II- und Natrium-salze der Diphosphonsäure enthalten, lagerfähig und über Monate verwendbar zu machen, werden sie, vorzugsweise in kleinen Flaschen abgefüllt, gefriergetrocknet und unter einem Schutzgas, wie z.B. Stickstoff, verschlossen.

Eine Markierungseinheit enthält vorzugsweise 1 bis 10 mg des lyophilisierten Gemisches.

Die erfindungsgemäß erhältlichen Markierungsbestecke werden nach Zusatz von Pertechnetat als Knochendiagnostika verwendet, wobei diese Präparate intravenös appliziert werden. Besonders bevorzugt sind daher solche Markierungsbestecke, die einen pH-Wert von 6.2 bis 7.2, vorzugsweise 6,5, aufweisen und neben dem Zinn-II-salz die Di- und Trinatriumsalze der Diphosphonsäure enthalten.

Außer einer optimalen und schnellen Knochenarreicherung zeigten die erfindungsgemäß erhältlichen Präparate im Tierversuch eine rasche Blutclearance sowie besonders geringe Leber- und Nierenspeicherungen.

Nach den bekannten Verfahren zur Herstellung von $Sn^{++}$-haltigen Diphosphonsäuren setzt man zu der sauren, wässrigen Lösung der Diphosphonsäure die saure, wässrige Lösung eines Zinn-II-salzes, vorwiegend $SnCl_2$, zu und neutralisiert an schließend. Solche Verfahren sind u.a. aus der GB—A—1 429 549 und der DE—A—25 43 350 bekannt.

Die Herstellung einer stabilen Tc-99m-Diphosphonsäure gemäß der Erfindung setzt demgegenüber voraus, daß wässrige Lösung der Diphosphonsäure vor Zugabe der Zinn-II-Verbindung neutralisiert wird.

Ein Vergleich der nach den bekannten Methoden hergestellten zinnhaltigen Diphosphonsäuren, markiert mit Tc-99m, mit solchen Diphosphonsäuren, die nach dem Verfahren gemäß der Erfindung hergestellt wurden, zeigt, daß sowohl in Bezug auf die Stabilität als auch auf die Organverteilung in der Ratte das Verfahren gemäß der Erfindung deutlich bessere Präparate liefert.

Beispiel 1

Zu einer wässrigen Lösung von 2 g Methylendiphosphonsäure gibt man 1 N NaOH bis ein pH-Wert von 6,5 erreicht ist. Der Lösung wird unter Ausschluß von Sauerstoff 50 mg reines Zinn-II-oxid zugesetzt. Bei Raumtemperatur wird gerührt, bis alles Zinn-II-oxid gelöst ist. Man füllt auf ein Gesamtvolumen von 100 ml Wasser auf, filtriert über ein Sterilfilter und füllt umgehend je 0.5 ml der Lösung unter Stickstoff als Schutzgas in vorgekühlte Gläschen ab. Nach der Gefriertrocknung enthält jede Markierungseinheit je 10 mg Methylendiphosphonsäure als Di-bzw. Trinatriumsalz mit je 2 Gewichtsprozent Zinn in Form von Zinn-II-Methylendiphosphonat.

Beispiel 2

32 mg Zinn-II-oxid werden in 14 ml 1 N Natronlauge gelöst und zu dieser Lösung unter Sauerstoffausschluß 36 ml einer wässrigen Lösung von 1 g Methylendiphosphonsäure gegeben, wobei ein pH von ca. 6,5 erreicht wird. Vereinzelung und Abfüllung erfolgen analog Beispiel 1.

Beispiel 3

Analog Beispiel 1 werden statt Methylendiphosphonsäure 2 g 1,1-Diphosphonopropan-2,3-dicarbonsäure eingesetzt. Die Umsetzung mit Zinn-II-oxid und die Vereinzelung der Natrium-Zinn-IIsalzlösung der 1,1-Diphosphono-propan-2,3-dicarbonsäure erfolgen wie dort beschrieben.

Beispiel 4

Ebenfalls analog Beispiel 2 setzt man 2 g 1,1-Diphosphonobutan-3,4-dicarbonsäure mit Zinn-II-oxid um und füllt wie dort angegeben ab.

**Patentansprüche**

1. Verfahren zur Herstellung eines zur Markierung mit Technetium-99m geeigneten Zinnsalzhaltigen Gemisches (Markierungsbesteck), dadurch gekennzeichnet, daß man Zinn-II-oxid entweder

a) mit dem Überschuß einer Natrium-Diphosphonatlösung, die einen pH-Wert zwischen 6,2 und 7,2 aufweist, umsetzt, oder
b) mit einem Überschuß an Natronlauge löst und soviel Diphosphonsäure zugibt, bis ein pH-Wert zwischen 6,2 und 7,2 erreicht ist.

2. Verfahren nach Anspruch 1, dadurch gekennzeichnet, daß die Reaktionstemperatur zwischen 20 und 100°C liegt.

3. Verfahren nach Ansprüchen 1 und 2, dadurch gekennzeichnet daß man unter Ausschluß von Sauerstoff arbeitet.

4. Verfahren nach Ansprüchen 1 bis 3, dadurch gekennzeichnet, daß man als Diphosphonsäure die Methylendiphosphonsäure, die 1,1-Diphosphonopropan-2,3-dicarbonsäure oder die 1,1-Diphosphonobutan-3,4-dicarbonsäure einsetzt.

**Revendications**

1. Procédé de préparation d'un mélange contenant un sel d'étain approprié au marquage avec le technétium-99m (Composition de marquage), caractérisé en ce que de l'oxyde d'étain (II)

a) est amené à réagir avec un excès d'une solution de diphosphonate de sodium qui a une valeur de pH comprise entre 6,2 et 7,2.
b) est dissous dans un excès de lessive de soude et on ajoute une quantité d'acide diphosphonique telle qu'elle permet d'atteindre une valeur de pH comprise entre 6,2 et 7,2.

2. Procédé selon la revendication 1, caractérisé en ce que la température de réaction est comprise entre 20 et 100°C.

3. Procédé selon la revendication 1 ou 2, caractérisé en ce qu'on travaille à l'abri de l'oxygène.

4. Procédé selon l'une quelconque des revendications 1 à 3 caractérisé en ce que, comme acide diphosphonique, on utilise l'acide méthylène diphosphonique, l'acide 1,1-diphosphonopropane-2,3-dicarboxylique ou l'acide 1,1-diphosphonobutane-3,4-dicarboxylique.

**Claims**

1. A process for the preparation of compositions labelling containing a mixture of technetium-99m and a tin salt, wherein tin-II-oxide is either

a) reacted with an excess of a sodium diphosphonate solution having a pH value of between 6.2 and 7.2, or
b) is dissolved in an excess of sodium hydroxide solution to which solution diphosphonic acid is added until a pH value of between 6.2 and 7.2 is obtained.

2. Process according to claim 1, wherein the reaction temperature is between 20 and 100°C.

3. Process according to claim 1 or 2, wherein the access of oxygen is prevented.

4. Process according to claim 1, 2 or 3, wherein the diphosphonic acid used is methylene-diphosphonic acid, 1,1-diphosphonopropane-2,3-dicarboxylic acid or 1,1-diphosphonobutane-3,4-dicarboxylic acid.